# EUROPEAN PATENT SPECIFICATION

(11) **EP 2 111 431 B1**
(45) Date of publication and mention of the grant of the patent: **01.05.2013**
(21) Application number: 07867828.1
(22) Date of filing: 18.12.2007
(51) Int. Cl.: C09K 5/04

(54) **PENTAFLUOROETHANE, TETRAFLUOROETHANE AND HYDROCARBON COMPOSITIONS**
PENTAFLUORETHAN-, TETRAFLUORETHAN- UND KOHLENWASSERSTOFF-ZUSAMMENSETZUNGEN
COMPOSITIONS DE PENTAFLUOROÉTHANE, DE TÉTRAFLUOROÉTHANE ET D'HYDROCARBURES

(30) Priority: 21.12.2006 US 876406 P
(43) Date of publication of application: 28.10.2009
(73) Proprietor: E. I. Du Pont de Nemours and Company, Wilmington, DE 19898 (US)
(72) Inventor: BIVENS, Donald Bernard, Kennett Square, PA 19348 (US)
(74) Representative: Matthews, Derek Peter
(86) International application number: PCT/US2007/025919
(87) International publication number: WO 2008/079226

(56) References cited:
- EP-A- 0 779 352
- WO-A-00/56834
- WO-A-01/23491
- WO-A-01/23493

## Description

### Field of the Invention

The present invention relates to heat transfer compositions that contain pentafluoroethane and tetrafluoroethane, and more than one hydrocarbon.

### Background of the Invention

The cooling industry has been responding to environmental regulations by providing alternative refrigerants that do not deplete the ozone layer for almost a decade.

Many alternative refrigerant blends have been proposed that function well as refrigerants. But some of these blends have limitations with respect to the use of the conventional refrigeration lubricants, such as mineral oil. The hydrofluorocarbon blends are often immiscible with mineral oil thus requiring that a new lubricant be used. The new lubricants being predominantly polyol esters are expensive and hydrophilic. Additionally, when retrofitting existing equipment with a hydrofluorocarbon based refrigerant, removal of the original lubricant and time consuming and costly flushing of the system to remove residual lubricant is necessary.

Certain refrigerant blends containing hydrocarbons have been proposed for improving the miscibility with mineral oils. However, many of these hydrocarbon containing refrigerant blends may be flammable, either as originally formulated in the liquid or vapor phase or may produce flammable mixtures upon leakage from a cooling system or from refrigerant storage containers. Therefore, only those blends that have been found to be non-flammable are widely accepted. These blends often do not contain enough hydrocarbon to improve the miscibility with mineral oil to the degree necessary to allow their use together.

WO 01/23491 discloses a CFC-12 replacement refrigerant comprising pentafluoroethane, 1,1,1,2-tetrafluoroethane and a satuated hydrocarbon or mixture thereof. The minimum content of 1,1,1,2-tetrafluoroethane is 83% by weight.

There remains a need for alternative compositions useful as heat transfer compositions with a balance of properties including non-flammability, capacity to match the refrigerant being replaced, good energy efficiency and the ability to provide adequate oil return when using mineral oil to lubricate the compressor.

### DESCRIPTION OF THE INVENTION

Disclosed are compositions containing pentafluoroethane (R125, CF₃CHF₂), 1,1,1,2-tetrafluoroethane (R134a, CF₃CH₂F), and at least two hydrocarbons each having eight or fewer carbon atoms. In certain embodiments, the hydrocarbon components consist of n-butane (R600, CH₃CH₂CH₂CH₃) and n-pentane (R601, CH₃CH₂CH₂CH₂CH₃),.

In certain embodiments the disclosed compositions are azeotrope-like.

In some embodiments, the pentafluoroethane is from 13% to 20% by weight of the composition; in other embodiments, the R125 is from 15% to 20% by weight; in other embodiments, the R125 is from 17% to 20% by weight; and in still other embodiments, the R125 is from 15% to 18% by weight.

In some embodiments, the R134a is from 70% to 80% by weight of the composition; in other embodiments, the R134a is from 70% to 75% by weight; in still other embodiments, the R134a is from 70% to 73% by weight; in other embodiments, the R134a is from 75% to 80% by weight; and in still other embodiments, the R134a is from 77% to 80% by weight.

In some embodiments, the hydrocarbon is selected from those having between 4 and 8 carbon atoms. In other embodiments, the hydrocarbon is selected from butanes, pentanes, hexanes, heptanes, and octanes. In some embodiments one hydrocarbon is selected from a hydrocarbon that is an alkene, cycloalkane, or mixtures thereof.

In some embodiments, the hydrocarbon component of the described composition is from 1% to 6% by weight; in other embodiments, the hydrocarbon is from 1.5% to 5%; in other embodiments, the hydrocarbon includes from 1% to 3% by weight n-butane. In some embodiments, the hydrocarbon includes from 0.5% to 2% by weight n-pentane.

Also, described is a composition comprising 13 weight percent to 20 weight percent pentafluoroethane; 70 weight percent to 80 weight percent 1,1,1,2-tetrafluoroethane; and 1 weight percent to 6 weight percent total of a combination of hydrocarbons consisting of n-butane and n-pentane; and in some embodiments, these compositions are azeotropic or azeotropic-like.

In some embodiments, the composition comprises 13 weight percent to 20 weight percent pentafluoroethane; 70 weight percent to 80 weight percent 1,1,1,2-tetrafluoroethane; 1 weight percent to 3 weight percent n-butane; and 0.5 to 2 weight percent n-pentane; and in some embodiments, these compositions are azeotropic or azeotropic-like.

In some embodiments, the invention further contains, 1,1,1,2,3,3,3-heptafluoropropane (R227ea, CF₃CHFCF₃).

In some embodiments, the composition comprises 15 weight percent to 18 weight percent pentafluoroethane; 70 weight percent to 75 weight percent 1,1,1,2-tetrafluoroethane; 1 weight percent to 3 weight percent n-butane; 0.5 to 2 weight percent n-pentane; and 5 weight percent to 15 weight percent 1,1,1,2,3,3,3-heptafluoropropane; and in some embodiments, these compositions are azeotropic or azeotropic-like.

In other embodiment, the composition comprises 15 weight percent to 20 weight percent pentafluoroethane; 75 weight percent to 80 weight percent 1,1,1,2-tetrafluoroethane; 1 weight percent to 3 weight percent n-butane; and 0.5 to 2.0 weight percent n-pentane.

Further embodiments are compositions that comprise 17 weight percent to 20 weight percent pentafluoroethane; 77 weight percent to 80 weight percent 1,1,1,2-tetrafluoroethane; 1 weight percent to 3 weight percent n-butane; and 0.5 to 2.0 weight percent n-pentane.

In yet other embodiments, the compositions further comprise 1,1,1,2,3,3,3-heptafluoropropane. In certain embodiments, the compositions comprise 5 weight percent to 15 weight percent 1,1,1,2, 3,3, 3-heptafluoropropane.

In further embodiments, the compositions comprise 15 weight percent to 18 weight percent pentafluoroethane; 70 weight percent to 75 weight percent 1,1,1,2-tetrafluoroethane, 1 weight percent to 3 weight percent n-butane; 0.5 weight percent to 2 weight percent n-pentane; and 5 weight percent to 15 weight percent 1,1,1,2,3,3,3-heptafluoropropane,

In other embodiments the compositions comprise 15 weight percent to 18 weight percent pentafluoroethane; 70 weight percent to 75 weight percent 1,1,1,2-tetrafluoroethane; 1 weight percent to 3 weight percent n-butane; 0.5 weight percent to 2 weight percent n-pentane; and 9 weight percent to 11 weight percent 1,1,1,2,3,3,3-heptafluoropropane.

In yet other embodiments the compositions comprise essentially of 15 weight percent to 17 weight percent pentafluoroethane; 70 weight percent to 73 weight percent 1,1,1,2-tetrafluoroethane; 1 weight percent to 3 weight percent n-butane; 0.5 weight percent to 2 weight percent n-pentane; and 9 weight percent to 11 weight percent 1,1,1,2,3,3,3-heptafluoropropane.

In other embodiments, the disclosed compositions comprise azeotropic or azeotrope-like compositions comprising 15 weight percent to 18 weight percent pentafluoroethane, 70 weight percent to 75 weig ht percent 1,1,1,2-tetrafluoroethane, 1 weight percent to 3 weight percent n-butane, 0.5 to 2 weight percent n-pentane, and 5 weight percent to 15 weight percent 1,1,1,2,3,3,3-heptafluoropropane.

In some embodiments, the described composition may include up to 15% R227ea.

In some embodiments, the composition includes from 5% to 15% R227ea. In other embodiments, the composition includes from 7% to 11% by weight; in other embodiments, R227ea is from 9% to 11% by weight.

All of these compounds are commercially available and/or may be made by known processes.

In some embodiments, the compositions further contain one or more other components, including but not limited to lubricants, compatibilizers, dyes (which may be an ultra-violet dye), solubilizing agents, and mixtures thereof. In one embodiment, the composition includes a lubricant that is one or more lubricants selected from the group consisting of mineral oils, alkylbenzene lubricants, synthetic lubricants, polyalkylene glycols (PAGs), polyol esters (POEs), and fluorinated oils.

In some embodiments, refrigeration system additives may optionally be added, as desired, to heat transfer compositions as disclosed herein in order to enhance lubricity and system stability. These additives are generally known within the field of refrigeration compressor lubrication, and include anti wear agents, extreme pressure lubricants, corrosion and oxidation inhibitors, metal surface deactivators, free radical scavengers, foam control agents, and the like. In general, these additives are present only in small amounts relative to the overall lubricant composition. They are typically used at concentrations of from less than about 0.01 % to as much as about 3 % of each additive. These additives are selected on the basis of the individual system requirements. Some typical examples of such additives may include, but are not limited to, lubrication enhancing additives, such as alkyl or aryl esters of phosphoric acid and of thiophosphates. These include members of the triaryl phosphate family of EP lubricity additives, such as butylated triphenyl phosphates (BTPP), or other alkylated triaryl phosphate esters, e.g. Syn-0-Ad 8478 from Akzo Chemicals, tricrecyl phosphates and related compounds. Additionally, the metal dialkyl dithiophosphates (e.g. zinc dialkyl dithiophosphate or ZDDP, Lubrizol 1375) and other members of this family of chemicals may be used in compositions of the present invention. Other antiwear additives include natural product oils and assymetrical polyhydroxyl lubrication additives such as Synergol TMS (International Lubricants). Similarly, stabilizers such as anti oxidants, free radical scavengers, and water scavengers may be employed. Compounds in this category can include, but are not limited to, butylated hydroxy toluene (BHT) and epoxides.

The disclosed compositions have a variety of utilities as working fluids, which include uses in the liquid and gas phase, and such utilities may be as foaming agents, blowing agents, cleaning agents, expansion agents for polyolefins and polyurethanes, carrier fluids, aerosol propellants, gaseous dielectrics, polymerization media, buffing abrasive agents, displacement drying agents, fire extinguishing or suppression agents, heat transfer mediums (such as heat transfer fluids including refrigerants for use in refrigeration systems, refrigerators, freezers, air conditioning systems, walk-in coolers, heat pumps, water chillers, and the like).

As used herein, an azeotropic composition is a composition containing two or more components (e.g., refrigerants) whose equilibrium vapor and liquid phase compositions are the same at a given pressure. At this pressure, the slope of the temperature vs. composition curve equals zero, which mathematically is expressed as (dt/dx)ₚ = 0), which in turn implies the occurrence of a maximum, minimum or saddle point temperature. In some embodiments, azeotropic compositions, exhibit some segregation of components at other conditions of temperature and/or pressure. The extent of the segregation depends on the particular azeotropic composition and its application of use. In some embodiments, an azeotropic composition is a constant boiling liquid admixture of two or more substances that behaves as a single substance, in that the vapor, produced by partial evaporation or distillation of the liquid has the same composition as the liquid, i.e., the admixture distills without substantial composition change. Constant boiling compositions, which are characterized as azeotropic, exhibit either a maximum or a minimum boiling point, as compared with that of the non-azeotropic mixtures of the same substances.

As used herein, the term "azeotrope-like composition" also sometimes referred to as "near-azeotropic composition," means a constant boiling, or substantially constant boiling liquid admixture of two or more substances that behaves very similarly to an azeotropic composition but does not meet the exact definition of an azeotropic composition. In some embodiments, the azeotrope-like composition may be characterized in that the vapor produced by partial evaporation or distillation of the liquid has substantially the same composition as the liquid from which it was evaporated or distilled. That is, the admixture distills/refluxes without substantial composition change. In some embodiments, an azeotrope-like composition may be characterized in that the bubble point vapor pressure of the composition and the dew point vapor pressure of the composition at a particular temperature are substantially the same. In some embodiments, a composition is azeotrope-like if, after 50 weight percent of the composition is removed such as by evaporation or boiling off, the difference in vapor pressure between the original composition and the composition remaining is less than 10 percent.

As used herein, compatibilizers are compounds which improve solubility of the hydrofluorocarbon of the compositions in conventional refrigeration, air-conditioning, and heat pump equipment lubricants and thus improve oil return to the compressor. In some embodiments, the composition is used with a system lubricant to reduced oil-rich phase viscosity.

Flammability is a term used to mean the ability of a composition to ignite and/or propagate a flame. For heat transfer compositions, the lower flammability limit ("LFL") is the minimum concentration of the heat transfer composition that is capable of propagating a flame through a homogeneous mixture of the composition and air under test conditions specified in ASTM (American Society of Testing and Materials) E681-2001.

As used herein, "ultra-violet" dye is defined as a UV fluorescent composition that absorbs light in the ultra-violet or "near" ultra-violet region of the electromagnetic spectrum. The fluorescence produced by the UV fluorescent dye under illumination by a UV light that emits radiation with wavelength anywhere from 10 nanometer to 750 nanometer may be detected.

As used herein, mobile refrigeration apparatus or mobile air-conditioning apparatus refers to any refrigeration or air-conditioning apparatus incorporated into a transportation unit for the road, rail, sea or air. In addition, apparatus, which are meant to provide refrigeration or air-conditioning for a system independent of any moving carrier, known as "intermodal" systems, are included in the present invention. Such intermodal systems include "containers" (combined sea/land transport) as well as "swap bodies" (combined road and rail transport).

As used herein, the term "lubricant" means any material added to a compressor (and in contact with any heat transfer composition in use within any refrigeration, air-conditioning or heat pump apparatus) that provide lubrication to the compressor to prevent parts from seizing and thus compressor failure. In some embodiments, lubricants may be one or more selected from the group consisting of mineral oils, alkylbenzene lubricants, synthetic lubricants, polyalkylene glycols (PAGs), polyol esters (POEs), and fluorinated oils.

As used herein, heat transfer compositions, typically are compositions utilized to transfer, move or remove heat from one space, location, object or body to a different space, location, object or body by radiation, conduction, or convection. A heat transfer composition may function as a secondary coolant by providing means of transfer for cooling (or heating) from a remote refrigeration (or heating) system. Heat transfer compositions may also be used in heat pumps. In some systems, the heat transfer composition may remain in a constant state throughout the transfer process (i.e., not evaporate or condense). Alternatively, evaporative cooling processes may utilize heat transfer compositions as well.

As used herein, a heat source may be defined as any space, location, object or body from which it is desirable to transfer, move or remove heat. Examples of heat sources may be spaces (open or enclosed) requiring refrigeration or cooling, such as refrigerator or freezer cases in a supermarket, building spaces requiring air-conditioning, industrial water chillers or the passenger compartment of an automobile requiring air-conditioning. A heat sink may be defined as any space, location, object or body capable of absorbing heat. A vapor compression refrigeration system is one example of such a heat sink.

In some embodiments the compositions described above are used as the refrigerant in a heat transfer system selected from the group consisting of air conditioners, freezers, refrigerators, walk-in coolers, heat pumps, and mobile refrigerator and air condition applications and combinations thereof.

A refrigerant is a compound or mixture of compounds that function as a heat transfer composition in a cycle wherein the composition undergoes a phase change from a liquid to a gas and back to a liquid.

Cooling capacity (also referred to as refrigeration capacity) is a measure of the change in enthalpy of a refrigerant in an evaporator per pound of refrigerant circulated, i.e., the heat removed by the refrigerant in the evaporator per a given time. The refrigeration capacity is a measure of the ability of a refrigerant or heat transfer composition to produce cooling. Therefore, the higher the capacity the greater the cooling that may be produced.

Energy efficiency (EER) is a term describing the efficiency of a cooling or heating system based upon the energy consumed in use.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, use of "a" or "an" are employed to describe elements and components described herein. This is done merely for convenience and to give a general sense of the scope of the invention. This description should be read to include one or at least one and the singular also includes the plural unless it is obvious that it is meant otherwise.

Unless otherwise defined, all technical and scientific terms used herein have the same meaning as commonly understood by one of ordinary skill in the art to which this invention belongs. Although methods and materials similar or equivalent to those described herein can be used in the practice or testing of embodiments of the present invention, suitable methods and materials are described below. All publications, patent applications, patents, and other references mentioned herein are incorporated by reference in their entirety, unless a particular passage is cited. In case of conflict, the present specification, including definitions, will control. In addition, the materials, methods, and examples are illustrative only and not intended to be limiting.

In some embodiments, the disclosed compositions maintain their non-flammable properties even during an equipment (e.g., air-conditioner, heat pump, refrigerator, or other cooling or heating system) leakage scenario.

In some embodiments, the disclosed compositions provide greater oil return to the compressor when using conventional mineral oil, as compared to previously known compositions of hydrofluorocarbon compositions comprising hydrocarbons. The improved oil-return may be due to lower viscosity of the heat transfer composition/mineral oil rich phase within the heat transfer equipment (e.g., air-conditioner, heat pump, refrigerator, or other cooling or heating system).

Advantages to some embodiments include improved (reduced) lubricant viscosity and thus improved oil return in use in vapor compression cooling systems, superior miscibility with mineral oil while remaining below industry acceptable flammability performance.

In order to be classified by ASHRAE (American Society of Heating, Refrigerating and Air-Conditioning Engineers, Inc.) as non-flammable, a refrigerant must be non-flammable under the conditions of ASTM E681-01 as formulated in both the liquid and vapor phase as well as during a leakage scenarios.

In many applications new heat transfer compositions are useful as refrigerants and must provide at least comparable refrigeration performance (meaning cooling capacity and energy efficiency), as well as compressor discharge pressure and discharge temperature, as the refrigerant for which a replacement is being sought. Excessive compressor discharge temperatures may breakdown the lubricant in the compressor leading to compressor failure.

Compositions disclosed herein have been shown to match refrigeration performance for R12 (dichlorodifluoromethane), R134a, and R413A (ASHRAE designation for a blend of 88 weight percent R134a, 9 weight percent R218 (octafluoropropane), and 3 weight percent isobutane).

In certain embodiments the above described compositions are suitable as replacement heat transfer compositions, which may be, but are not limited to, R12, R134a, and R413A. R12, R134a, and R413A are often used in automotive air-conditioning systems, stationary air-conditioning systems, as well as direct expansion stationary medium temperature refrigeration systems, such as food service, supermarket display cases, food storage and processing, and domestic refrigerators or freezers. In general, many of the compositions described herein may be useful for any positive displacement compressor system designed for any number of heat transfer fluids, including refrigerants, including R12, R134a, and R413A. Additionally, many of the compositions may be useful in new equipment utilizing positive displacement compressors to provide similar performance to the aforementioned refrigerants. In some embodiments, the described compositions have unexpected improved performance in terms of the combined characteristics of non-flammability, refrigeration capacity, energy efficiency, and mineral oil viscosity reduction.

Also described herein is a process to produce cooling comprising condensing a composition as disclosed herein and thereafter evaporating said composition in the vicinity of a body to be cooled.

In some embodiments, the use of the above described compositions includes using the composition as a heat transfer composition in a process to produce heat comprising condensing a composition as disclosed herein in the vicinity of a body to be heated and thereafter evaporating said composition.

In some embodiments, the use of the above described compositions includes using the composition as a heat transfer composition in a process for producing cooling, wherein the composition is first cooled and stored under pressure and when exposed to a warmer environment, the composition absorbs some of the ambient heat, expands, and the warmer environment is thusly cooled.

A method for recharging a cooling or heating system that contains a refrigerant to be replaced and a lubricant, said method comprising removing the refrigerant to be replaced from the cooling or heating system while retaining a substantial portion of the lubricant in said system, and introducing to the cooling or heating system a composition as disclosed herein.

### EXAMPLES

The concepts described herein will be further described in the following examples, which do not limit the scope of the invention described in the claims.

### Impact of Vapor Leakage

A vessel is charged with an initial composition at a temperature of 25 °C, and the initial vapor pressure of the composition is measured. The composition is allowed to leak from the vessel, while the temperature is held constant, until 50 weight percent of the initial composition is removed, at which time the vapor pressure of the composition remaining in the vessel is measured. Calculated results are shown in Table 1.

**TABLE 1**

| **Composition (weight percent)** | **Initial Pressure (Psia)** | **Initial Pressure (kPa)** | **Pressure After 50% Leak (Psia)** | **Pressure After 50% Leak (kPa)** | **Change in pressure (%)** |
|---|---|---|---|---|---|
| R125/R134a/n-butane/n-pentane | | | | | |
| 15/80/3/2 | 111.9 | 771.5 | 106.4 | 733.6 | 4.9 |
| 16/80/2/2 | 112 | 772.2 | 106.2 | 732.2 | 5.2 |
| 18/79/2/1 | 114.5 | 789.4 | 108.3 | 746.7 | 5.4 |
| 18/79/1.5/1.5 | 113.6 | 783.2 | 107.4 | 740.5 | 5.5 |
| 19/78/1.8/1.2 | 115 | 792.9 | 108.6 | 748.8 | 5.6 |
| 20/75/3/2 | 116.2 | 801.2 | 109.6 | 755.7 | 5.7 |
| 20/77/2/1 | 116.2 | 801.2 | 109.6 | 755.7 | 5.7 |
| 18/80/1.5/0.5 | 114.3 | 788.1 | 108.1 | 745.3 | 5.4 |
| 20/78/1.5/0.5 | 116.1 | 800.5 | 109.4 | 754.3 | 5.8 |
| 20/78.5/1/0.5 | 115.5 | 796.3 | 108.9 | 750.8 | 5.7 |
| 20/77.5/2/0.5 | 116.6 | 803.9 | 110.0 | 758.4 | 5.7 |

| R125/R134a/R227ea/n-butane/n-pentane | | | | | |
|---|---|---|---|---|---|
| 15/72/10/2/1 | 111.1 | 766.0 | 105.1 | 724.6 | 5.4 |
| 15/74/9/1/1 | 110.1 | 759.1 | 104.3 | 719.1 | 5.3 |
| 15/78/5/1/1 | 110.4 | 761.2 | 104.8 | 722.6 | 5.1 |
| 16/66/15/2/1 | 111.6 | 769.5 | 105 | 723.9 | 5.9 |
| 18/70/9/2/1 | 113.9 | 785.3 | 107.2 | 739.1 | 5.9 |
| 15/74.5/9/1/0.5 | 110.4 | 761.2 | 104.6 | 721.2 | 5.3 |
| 17.5/70/11/1/0.5 | 112.6 | 776.3 | 106.0 | 730.8 | 5.9 |
| 17/70/11/1.5/0.5 | 112.7 | 777.0 | 106.2 | 732.2 | 5.8 |

The difference in vapor pressure between the original composition and the composition remaining after 50 weight percent is removed is less then about 10 percent for compositions as disclosed herein. This indicates that compositions as disclosed herein are azeotropic or azeotrope-like compositions.

### Lubricant viscosity

Viscosity of the compositions disclosed herein combined with mineral oil may be determined . A heat transfer composition is combined with 5 weight % Suniso 3GS mineral oil and the mixed composition is flashed such that the vapor phase occupies 20 % of the total volume (somewhat like a typical system receiver). The liquid phase (oil-rich phase) viscosity (in cp = centipoise) for a heat transfer composition of the present invention is shown in Table 2.

**TABLE 2**

| Mixture composition (wt%) | | | | | Oil-rich phase viscosity (cp) |
|---|---|---|---|---|---|
| R125 | R134a | n-butane | n-pentane | isopentane | |
| 19.0 | 78.0 | 2.0 | 1.0 | 0 | 2405 |
| 19.0 | 78.0 | 2.0 | 0 | 1.0 | 2963 |
| | | | | | |
| 5.0 | 93.0 | 1.4 | 0.6 | 0 | 5067 |
| 5.0 | 93.0 | 1.4 | 0 | 0.6 | 5781 |

It can be seen that the compositions above containing n-pentane have lower oil-rich phase viscosity than the compositions containing isopentane. It is expected that the n-pentane compositions will provide improved oil return when compared to the isopentane compositions in use in a refrigeration, air-conditioning, or heat-pump system.

### Refrigeration Performance Data

Table 3 shows the calculated performance characteristics of various heat transfer compositions as disclosed herein and compared to the same measured performance characteristics for R12 and R134a.

In Table 3, Comp Discharge Temp is compressor discharge temperature, Comp Discharge Pres is compressor discharge pressure, and EER is energy efficiency. The data are based on the following conditions.

| | |
|---|---|
| Evaporator temperature | 33.0°F (0.56°C) |
| Condenser temperature | 140.0°F (60.0°C) |
| Return gas temperature | 42.0°F (5.6°C) |
| Compressor efficiency is | 80% |

Note that the superheat is included in cooling capacity calculations. Super heat is a term used to define the amount of heat added to a vapor compositions above its saturation vapor temperature.

**TABLE 3**

| **Samp** | **Composition** | **Cooling capacity (BTU/ft³)** | **Cooling capacity (kJ/m³)** | **EER** | **Comp Discharge Pres (psia)** | **Comp Discharge Pres (kPa)** | **Comp Discharge Temp (F)** | **Comp Discharge Temp (C)** |
|---|---|---|---|---|---|---|---|---|
| | **Comparative** | | | | | | | |
| A | R12 | 45.2 | 1683 | 7.1 | 221 | 1525 | 181 | 82.8 |
| B | R134a | 44.1 | 1642 | 6.8 | 244 | 1685 | 174 | 78.9 |
| C | R413A | 45.5 | 1694 | 6.4 | 268 | 1848 | 169 | 76.1 |
| D | R125/R134a/R32/n-pentane (20/38/40/2 wt%) | 82.0 | 3053 | 7.2 | 385 | 2654 | 199 | 92.8 |
| | | | | | | | | |
| | *Selected Embodiments of the composition described herein* | | | | | | | |
| 1 | R125/R134a/n-butane/n-pentane (14/80/3/3 wt%) | 45.1 | 1679 | 6.5 | 262 | 1806 | 168 | 75.6 |
| 2 | R125/R134a/n-butane/n-pentane (14/80/2/4 wt%) | 44.1 | 1672 | 6.4 | 262 | 1779 | 169 | 76.1 |
| 3 | R125/R134a/n-butane/n-pentane (14/80/4/2 wt%) | 45.7 | 1702 | 6.4 | 265 | 1827 | 169 | 76.1 |
| 4 | R125/R134a/n-butane/n-pentane (15/79/3/3 wt%) | 45.3 | 1687 | 6.4 | 263 | 1813 | 168 | 75.6 |
| 5 | R125/R134a/n-butane/n-pentane (15/79/2/4 wt%) | 44.1 | 1642 | 6.4 | 259 | 1786 | 169 | 76.1 |
| 6 | R125/R134a/n-butane/n-pentane (15/79/4/2 wt%) | 45.8 | 1705 | 6.4 | 267 | 1841 | 168 | 75.6 |
| 7 | R125/R134a/n-butane/n-pentane (15/80/2.5/2.5 wt%) | 45.6 | 1698 | 6.5 | 264 | 1820 | 169 | 76.1 |
| 8 | R125/R134a/n-butane/n-pentane (15/79/3/2 wt%) | 45.9 | 1709 | 6.4 | 266 | 1834 | 169 | 76.1 |
| 9 | R125/R134a/n-butane/n-pentane (16/78/3/3 wt%) | 45.4 | 1690 | 6.4 | 264 | 1820 | 168 | 75.6 |
| 10 | R125/R134a/n-butane/n-pentane (16/80/2/2 wt%) | 46.0 | 1713 | 6.5 | 267 | 1841 | 170 | 76.7 |
| 11 | R125/R134a/n-butane/n-pentane (17/80/2/1 wt%) | 46.5 | 1731 | 6.4 | 271 | 1869 | 170 | 76.7 |
| 12 | R125/R134a/n-butane/n-pentane (17/79/2/2 wt%) | 46.1 | 1716 | 6.4 | 268 | 1848 | 170 | 76.7 |
| 13 | R125/R134a/n-butane/n-pentane (17/78/3/2 wt%) | 46.1 | 1716 | 6.4 | 269 | 1855 | 169 | 76.1 |
| 14 | R125/R134a/n-butane/n-pentane (18/76/3/3 wt%) | 45.9 | 1709 | 6.4 | 267 | 1841 | 171 | 77.2 |
| 15 | R125/R134a/n-butane/n-pentane (18/76/2/4 wt%) | 43.7 | 1627 | 6.3 | 263 | 1813 | 170 | 76.7 |
| 16 | R125/R134a/n-butane/n-pentane (18/76/4/2 wt%) | 46.2 | 1720 | 6.4 | 271 | 1869 | 168 | 75.6 |
| 17 | R125/R134a/n-butane/n-pentane (18/77/3/3 wt%) | 46.2 | 1720 | 6.4 | 271 | 1869 | 169 | 76.1 |
| 18 | R125/R134a/n-butane/n-pentane (18/78/2/2 wt%) | 46.2 | 1720 | 6.4 | 270 | 1862 | 170 | 76.7 |
| 19 | R125/R134a/n-butane/n-pentane (18/78/2/2 wt%) | 46.7 | 1739 | 6.4 | 274 | 1889 | 170 | 76.7 |
| 20 | R125/R134a/n-butane/n-pentane (18/79/2/1 wt%) | 46.7 | 1739 | 6.4 | 273 | 1882 | 170 | 76.7 |
| 21 | R125/R134a/n-butane/n-pentane (18/79/1.5/1.5 wt%) | 46.5 | 1731 | 6.4 | 271 | 1869 | 170 | 76.7 |
| 22 | R125/R134a/n-butane/n-pentane (18/80/1/1 wt%) | 46.6 | 1735 | 6.4 | 271 | 1869 | 171 | 77.2 |
| 23 | R125/R134a/n-butane/n-pentane (18.5/80/1/0.5 wt%) | 46.9 | 1746 | 6.4 | 273 | 1882 | 171 | 77.2 |
| 24 | R125/R134a/n-butane/n-pentane (19/76.5/2/2.5 wt%) | 46.1 | 1716 | 6.4 | 270 | 1862 | 169 | 76.1 |
| 25 | R125/R134a/n-butane/n-pentane (19/77/2/2 wt%) | 46.4 | 1728 | 6.4 | 271 | 1869 | 170 | 76.7 |
| 26 | R125/R134a/n-butane/n-pentane (19/78/2/1 wt%) | 46.8 | 1743 | 6.4 | 274 | 1889 | 170 | 76.7 |
| 27 | R125/R134a/n-butane/n-pentane (19/78/1.8/1.2 wt%) | 46.7 | 1739 | 6.4 | 273 | 1882 | 170 | 76.7 |
| 28 | R125/R134a/n-butane/n-pentane (19/78/1.5/1.5 wt%) | 46.6 | 1735 | 6.4 | 272 | 1875 | 170 | 76.7 |
| 29 | R125/R134a/n-butane/n-pentane (19/78/1/2 wt%) | 46.4 | 1728 | 6.4 | 270 | 1862 | 170 | 76.7 |
| 30 | R125/R134a/n-butane/n-pentane (19/79/1/1 wt%) | 46.8 | 1743 | 6.4 | 273 | 1882 | 171 | 77.2 |
| 31 | R125/R134a/n-butane/n-pentane (19.5/79/1/0.5 wt%) | 47.0 | 1750 | 6.4 | 275 | 1896 | 171 | 77.2 |
| 32 | R125/R134a/n-butane/n-pentane (20/74/3/3 wt%) | 45.8 | 1705 | 6.4 | 270 | 1862 | 168 | 75.6 |
| 33 | R125/R134a/n-butane/n-pentane (20/74/2/4 wt%) | 43.3 | 1612 | 6.2 | 266 | 1834 | 171 | 77.2 |
| 34 | R125/R134a/n-butane/n-pentane (20/74/4/2 wt%) | 46.5 | 1731 | 6.3 | 274 | 1889 | 168 | 75.6 |
| 35 | R125/R134a1n-butane/n-pentane (20/75/3/2 wt%) | 46.5 | 1731 | 6.4 | 274 | 1889 | 169 | 76.1 |
| 36 | R125/R134a/n-butane/n-pentane (20/76/2/2 wt%) | 46.5 | 1731 | 6.4 | 273 | 1882 | 169 | 76.1 |
| 37 | R125/R134a/n-butane/n-pentane (20/77/2/1 wt%) | 46.9 | 1746 | 6.4 | 276 | 1903 | 170 | 76.7 |
| 38 | R125/R134a/n-butane/n-pentane (20/77/1.5/1.5 wt%) | 46.7 | 1739 | 6.4 | 274 | 1889 | 170 | 76.7 |
| 39 | R125/R134a/n-butane/n-pentane (20/78/1/1 wt%) | 46.9 | 1746 | 6.4 | 274 | 1889 | 171 | 77.2 |
| 40 | R125/R134a/n-butane/n-pentane (20/78.5/1/0.5 wt%) | 47.1 | 1754 | 6.4 | 275 | 1896 | 171 | 77.2 |

In some embodiments, the newly described compositions above have substantially matching or even higher cooling capacity than R12, R134a, and/or R413A while maintaining lower discharge temperatures and pressures. The EER (energy efficiency) for these compositions is also within about 10% or better as compared to R12, R134a, and/or R413A. This indicates that these compositions could be replacement refrigerants for R12, R134a, or R413A, in refrigeration, air-conditioning or heat pump cooling/heating equipment. Sample D, by comparison does not provide matching performance and would not make a good drop-in replacement for these heat transfer compositions due to higher system pressures.

### Refrigeration Performance Data

Table 4 shows the calculated performance characteristics of various heat transfer compositions as disclosed herein and compared to the same measured performance characteristics for R12 and R134a.

In Table 4, Comp Discharge Temp is compressor discharge temperature, Comp Discharge Pres is compressor discharge pressure, and EER is energy efficiency. The data are based on the following conditions.

| | |
|---|---|
| Evaporator temperature | 33.0°F (0.56°C) |
| Condenser temperature | 140.0°F (60.0°C) |
| Return gas temperature | 42.0°F (5.6°C) |
| Compressor efficiency is | 80% |

| | |
|---|---|
| Note that the superheat is included in cooling capacity calculations. | |

**TABLE 4**

| | **Composition** | **Cooling capacity (BTU/ft³)** | **Cooling capacity (kJ/m³)** | **EER** | **Comp Discharge Pres (psia)** | **Comp Discharge Pres (kPa)** | **Comp Discharge Temp (F)** | **Comp Discharge Temp (C)** |
|---|---|---|---|---|---|---|---|---|
| | **Comparative** | | | | | | | |
| A | R12 | 45.2 | 1683 | 7.1 | 221 | 1525 | 181 | 82.8 |
| B | R134a | 44.1 | 1642 | 6.8 | 244 | 1685 | 174 | 78.9 |
| C | R413A | 45.5 | 1694 | 6.4 | 268 | 1848 | 169 | 76.1 |
| D | R125/R134a/R227ea/R32 (10/50/10/30 wt%) | 67.3 | 2505 | 6.4 | 380 | 2620 | 203 | 95.0 |
| E | R125/R134a/R227ea (30/20/50 wt%) | 39.4 | 1467 | 5.8 | 269 | 1855 | 152 | 66.7 |
| F | R125/R134a/R227ea (20/20/60 wt% | 36.5 | 1359 | 5.9 | 245 | 1689 | 145 | 62.8 |
| | | | | | | | | |
| | *Selected Embodiments of the composition described herein* | | | | | | | |
| 1 | R125/R134a/R227ea/n-butane/n-pentane (15/71/9/3/2 wt%) | 44.8 | 1668 | 6.4 | 264 | 1820 | 166 | 74.4 |
| 2 | R125/R134a/R227ea/n-butane/n-pentane (15/72/9/3/1 wt%) | 45.3 | 1687 | 6.4 | 267 | 1841 | 166 | 74.4 |
| 3 | R125/R134a/R227ea/n-butane/n-pentane (15/73/9/2/1 wt%) | 45.3 | 1687 | 6.4 | 266 | 1834 | 167 | 75.0 |
| 4 | R125/R134a/R227ea/n-butane/n-pentane (15/73/9/1/1.5 wt%) | 45.1 | 1679 | 6.4 | 264 | 1820 | 167 | 75.0 |
| 5 | R125/R134a/R227ea/n-butane/n-pentane (15.5/73/9/1/1.5 wt%) | 45.1 | 1679 | 6.4 | 264 | 1820 | 167 | 75.0 |
| 6 | R125/R134a/R227ea/n-butane/n-pentane (15/74/9/1/1 wt%) | 45.2 | 1683 | 6.5 | 265 | 1827 | 168 | 75.6 |
| 7 | R125/R134a/R227ea/n-butane/n-pentane (15/74.5/9/1/0.5 wt%) | 45.5 | 1694 | 6.5 | 266 | 1834 | 168 | 75.6 |
| 8 | R125/R134a/R227ea/n-butane/n-pentane (15.5/74/9/1/0.5 wt%) | 45.5 | 1694 | 6.4 | 267 | 1841 | 168 | 75.6 |
| 9 | R125/R134a/R227ea/n-butane/n-pentane (16/71/9/2/2 wt%) | 44.9 | 1672 | 6.4 | 265 | 1827 | 166 | 74.4 |
| 10 | R125/R134a/R227ea/n-butane/n-pentane (16/70/6/3/2 wt%) | 44.9 | 1672 | 6.4 | 265 | 1827 | 166 | 74.4 |
| 11 | R125/R134a/R227ea/n-butane/n-pentane (17/71/9/1.5/1.5 wt%) | 45.3 | 1687 | 6.4 | 267 | 1841 | 167 | 75.0 |
| 12 | R125/R134a/R227ea/n-butane/n-pentane (17/70/9/2/2 wt%) | 45.1 | 1679 | 6.4 | 266 | 1834 | 166 | 74.4 |
| 13 | R125/R134a/R227ea/n-butane/n-pentane (18/70/9/1.5/1.5 wt%) | 45.4 | 1690 | 6.4 | 269 | 1855 | 167 | 75.0 |
| 14 | R125/R134a/R227ea/n-butane/n-pentane (18/70/9/1/2 wt%) | 45.2 | 1683 | 6.4 | 267 | 1841 | 167 | 75.0 |
| 15 | R125/R134a/R227ea/n-butane/n-pentane (18/71/9/1/1 wt%) | 45.6 | 16998 | 6.4 | 269 | 1855 | 167 | 75.0 |
| 16 | R125/R134a/R227ea/n-butane/n-pentane (15/71/10/2/2 wt%) | 44.7 | 1664 | 6.4 | 263 | 1813 | 166 | 74.4 |
| 17 | R125/R134a/R227ea/n-butane/n-pentane (15/72/10/2/1 wt%) | 45.2 | 1683 | 6.4 | 266 | 1834 | 167 | 75.0 |
| 18 | R125/R134a/R227ea/n-butane/n-pentane (15/72/10/1/2 wt%) | 44.7 | 1664 | 6.4 | 262 | 1806 | 166 | 74.4 |
| 19 | R125/R134a/R227ea/n-butane/n-pentane (16/70/10/2/2 wt%) | 44.8 | 1668 | 6.4 | 264 | 1820 | 166 | 74.4 |
| 20 | R125/R134a/R227ea/n-butane/n-pentane (16/71/10/2/1 wt%) | 45.3 | 1687 | 6.4 | 267 | 1841 | 167 | 75.0 |
| 21 | R125/R134a/R227ea/n-butane/n-pentane (17/71/10/1/1 wt%) | 45.4 | 1690 | 6.4 | 268 | 1848 | 167 | 75.0 |
| 22 | R125/R134a/R227ea/n-butane/n-pentane (18/70/10/1/1 wt%) | 45.5 | 1694 | 6.4 | 269 | 1855 | 167 | 75.0 |
| 23 | R125/R134a/R227ea/n-butane/n-pentane (15/70/11/3/1 wt%) | 45.0 | 1676 | 6.4 | 266 | 1834 | 166 | 74.4 |
| 24 | R125/R134a/R227ea/n-butane/n-pentane (15/70/11/2/3 wt%) | 44.6 | 1660 | 6.4 | 262 | 1806 | 165 | 73.9 |
| 25 | R125/R134a/R227ea/n-butane/n-pentane (15/71/11/2/1 wt%) | 45.0 | 1676 | 6.4 | 265 | 1827 | 166 | 74.4 |
| 26 | R125/R134a/R227ea/n-butane/n-pentane (15/71/11/1.5/1.5 wt%) | 44.8 | 1668 | 6.4 | 263 | 1813 | 166 | 74.4 |
| 27 | R125/R134a/R227ea/n-butane/n-pentane (16/70/11/2/1 wt%) | 45.2 | 1683 | 6.4 | 267 | 1841 | 166 | 74.4 |
| 28 | R125/R134a/R227ea/n-butane/n-pentane (16/70/11/1.5/1.5 wt%) | 44.9 | 1672 | 6.4 | 265 | 1827 | 166 | 74.4 |
| 29 | R125/R134a/R227ea/n-butane/n-pentane (17/70/11/1/1 wt%) | 45.3 | 1687 | 6.4 | 267 | 1841 | 167 | 75.0 |
| 30 | R125/R134a/R227ea/n-butane/n-pentane (17.5/70/11/1/0.5 wt%) | 45.5 | 1694 | 6.4 | 269 | 1855 | 167 | 75.0 |
| 31 | R125/R134a/R227ea/n-butane/n-pentane (18/73.5/5/2/1.5 wt%) | 45.9 | 1709 | 6.4 | 270 | 1862 | 168 | 75.6 |
| 32 | R125/R134a/R227ea/n-butane/n-pentane (18/74/5/2/1 wt%) | 46.1 | 1716 | 6.4 | 272 | 1875 | 168 | 75.6 |
| 33 | R125/R134a/R227ea/n-butane/n-pentane (18/74/5/2.5/0.5 wt%) | 46.4 | 1728 | 6.4 | 274 | 1889 | 169 | 75.6 |
| 34 | R125/R134a/R227ea/n-butane/n-pentane (18/71/7/2/2 wt%) | 45.4 | 1690 | 6.4 | 268 | 1848 | 167 | 75.0 |
| 35 | R125/R134a/R227ea/n-butane/n-pentane (15/68/13/2/2 wt%) | 44.3 | 1649 | 6.4 | 262 | 1806 | 165 | 73.9 |
| 36 | R125/R134a/R227ea/n-butane/n-pentane (15/66/15/2/2 wt%) | 44.0 | 1638 | 6.4 | 261 | 1800 | 164 | 73.3 |

Many compositions of the present invention have matching or even higher cooling capacity than R12, R134a, and/or R413A while maintaining similar discharge temperatures and pressures. The EER (energy efficiency) for these compositions is also within about 10% or better as compared to R12, R134a, and/or R413A. This indicates that these compositions could be replacement refrigerants for R12, R134a, or R413A, in refrigeration, air-conditioning or heat pump cooling/heating equipment. Sample D, by comparison does not provide matching performance and would not make a good replacement for these heat transfer compositions. Additionally, Samples E and F have cooling capacities and EER considerably lower than R12, R134a, and R413A, therefore, also not making good replacement compositions for these refrigerants.

### Calorimeter testing

Laboratory calorimeter tests at 3 evaporator temperatures were conducted using a heat transfer composition containing 19 weight percent R125, 78 weight percent R134a, 1.8 weight percent n-butane, and 1.2 weight percent n-pentane. Pure R134a was also tested for direct comparison. An Embraco EMU 60 HSC positive displacement compressor was used and the conditions for the tests were as follows:

| | |
|---|---|
| Condenser temperature : | 130 °F (54.4 °C) |
| Liquid temperature: | 90 °F (32.2 °C) |
| Suction temperature: | 90 °F (32.2 °C) |
| Evaporator temperatures: | -13, -4, and +14 °F (-25, -20, and -10 °C) |

Results are listed in Table 5.

**TABLE 5**

| | **Evap. Temp. -13 F** | | **Evap. Temp. -4 F** | | **Evap. Temp. +14 F** | |
|---|---|---|---|---|---|---|
| | | **(-25 C)** | | **(-20 C)** | | **(-10 C)** |
| | **R134a** | **R125/R134a/ n-butane/ n-pentane** | **R134a** | **R125/R134a/ n-butane/ n-pentane** | **R134a** | **R125/R134a/ n-butane/ n-pentane** |
| Cooling Capacity, Btu/hr | 504 | 544 | 689 | 761 | 1145 | 1169 |
| Energy Efficiency, EER | 4.4 | 4.4 | 5.1 | 5.2 | 6.6 | 6.3 |
| Discharge Press., psia (kPa) | 215.7 (1487) | 235.7 (1625) | 215.7 (1487) | 235.7 (1625) | 215.7 (1487) | 235.7 (1625) |
| Suction Press., psia (kPa) | 15.4 (106) | 16.7 (115) | 19.3 (133) | 21.7 (150) | 29.1 (201) | 31.7 (219) |
| Compressor shell exit Temp., F (°C) | 130 (54.4) | 129 (53.9) | 131 (55.0) | 131 (55.0) | 139 (59.4) | 140 (60.0) |

It can be seen from the above data that the compositions as disclosed herein provide similar performance to R134a in use and therefore may serve as a replacement for R134a. In some embodiments, it is expected that no major equipment modifications will be necessary.

Note that not all of the activities described above in the general description or the examples are required, that a portion of a specific activity may not be required, and that one or more further activities may be performed in addition to those described. Still further, the order in which activities are listed are not necessarily the order in which they are performed.

In the foregoing specification, the concepts have been described with reference to specific embodiments. However, one of ordinary skill in the art appreciates that various modifications and changes can be made without departing from the scope of the invention as set forth in the claims below. Accordingly, the specification are to be regarded in an illustrative rather than a restrictive sense, and all such modifications are intended to be included within the scope of invention.

Benefits, other advantages, and solutions to problems have been described above with regard to specific embodiments. However, the benefits, advantages, solutions to problems, and any feature(s) that may cause any benefit, advantage, or solution to occur or become more pronounced are not to be construed as a critical, required, or essential feature of any or all the claims.

It is to be appreciated that certain features are, for clarity, described herein in the context of separate embodiments, may also be provided in combination in a single embodiment. Conversely, various features that are, for brevity, described in the context of a single embodiment, may also be provided separately or in any subcombination. Further, reference to values stated in ranges include each and every value within that range.

## Claims

1. A composition comprising:
13 weight percent to 20 weight percent pentafluoroethane;
70 weight percent to 80 weight percent 1,1,1,2-tetrafluoroethane; and
1 weight percent to 6 weight percent total of a combination of hydrocarbons consisting of n-butane and n-pentane.

2. A composition of claim 1, wherein said combination of hydrocarbons comprises from 1 weight percent to 3 weight percent n-butane; and 0.5 weight percent to 2 weight percent n-pentane.

3. The composition of claim 1, comprising:
15 weight percent to 20 weight percent pentafluoroethane;
75 weight percent to 80 weight percent 1,1,1,2-tetrafluoroethane;
1 weight percent to 3 weight percent n-butane; and
0.5 weight percent to 2 weight percent n-pentane.

4. The composition of claim 1, comprising:
17 weight percent to 20 weight percent pentafluoroethane;
77 weight percent to 80 weight percent 1,1,1,2-tetrafluoroethane;
1 weight percent to about 3 weight percent n-butane; and
0.5 to 2 weight percent n-pentane.

5. The composition of claim 1, further comprising 1,1,1,2,3,3,3-heptafluoropropane.

6. The composition of claim 5, further comprising 5 weight percent to 15 weight percent 1,1,1,2,3,3,3-heptafluoropropane.

7. The composition of claim 6, comprising:
15 weight percent to 18 weight percent pentafluoroethane;
70 weight percent to 75 weight percent 1,1,1,2-tetrafluoroethane;
1 weight percent to 3 weight percent n-butane;
0.5 to 2 weight percent n-pentane; and
5 weight percent to 15 weight percent 1,1,1,2,3,3,3-heptafluoropropane.

8. The composition of claim 6, comprising:
15 weight percent to 17 weight percent pentafluoroethane;
70 weight percent to 73 weight percent 1,1,1,2-tetrafluoroethane;
1 weight percent to 3 weight percent n-butane;
0.5 weight percent to 2 weight percent n-pentane; and
9 weight percent to 11 weight percent 1,1,1,2,3,3,3-heptafluoropropane.

9. The composition of claim 1 comprising an azeotrope-like composition comprising:
13 weight percent to 20 weight percent pentafluoroethane;
70 weight percent to 80 weight percent 1,1,1,2-tetrafluoroethane;
1 weight percent to 3 weight percent n-butane; and
0.5 to 2 weight percent n-pentane.

10. The composition of claim 5 comprising an azeotrope-like composition comprising:
15 weight percent to 18 weight percent pentafluoroethane;
70 weight percent to 75 weight percent 1,1,1,2-tetrafluoroethane;
1 weight percent to 3 weight percent n-butane;
0.5 to 2 weight percent n-pentane; and
5 weight percent to 15 weight percent 1,1,1,2,3,3,3-heptafluoropropane.

11. A process to produce cooling comprising condensing the composition of claim 1 and thereafter evaporating said composition in the vicinity of a body to be cooled.

12. A process to produce heat comprising condensing the composition of claim 1 in the vicinity of a body to be heated and thereafter evaporating said composition.

13. A method for recharging a cooling system that contains a refrigerant to be replaced and a lubricant, said method comprising:
removing the refrigerant to be replaced from the cooling system while retaining a substantial portion of the lubricant in said system; and
introducing to the cooling system a refrigerant composition of claim 1.

14. A heat exchange system comprising a composition of claim 1, wherein said system is selected from the group consisting of air conditioners, freezers, refrigerators, water chillers, walk-in coolers, heat pumps, and mobile refrigerator and air conditioning applications and combinations thereof.

## Patentansprüche

1. Zusammensetzung umfassend:
13 Gewichtsprozent bis 20 Gewichtsprozent Pentafluorethan;
70 Gewichtsprozent bis 80 Gewichtsprozent 1,1,1,2-Tetrafluorethan; und
1 Gewichtsprozent bis 6 Gewichtsprozent insgesamt einer Kombination von Kohlenwasserstoffen, die aus n-Butan und n-Pentan besteht.

2. Zusammensetzung nach Anspruch 1, wobei die Kombination von Kohlenwasserstoffen 1 Gewichtsprozent bis 3 Gewichtsprozent n-Butan und 0,5 Gewichtsprozent bis 2 Gewichtsprozent n-Pentan umfasst.

3. Zusammensetzung nach Anspruch 1, umfassend:
15 Gewichtsprozent bis 20 Gewichtsprozent Pentafluorethan;
75 Gewichtsprozent bis 80 Gewichtsprozent 1,1,1,2-Tetrafluorethan;
1 Gewichtsprozent bis 3 Gewichtsprozent n-Butan; und
0,5 Gewichtsprozent bis 2 Gewichtsprozent n-Pentan.

4. Zusammensetzung nach Anspruch 1, umfassend:
17 Gewichtsprozent bis 20 Gewichtsprozent Pentafluorethan;
77 Gewichtsprozent bis 80 Gewichtsprozent 1,1,1,2-Tetrafluorethan;
1 Gewichtsprozent bis etwa 3 Gewichtsprozent n-Butan; und
0,5 Gewichtsprozent bis 2 Gewichtsprozent n-Pentan.

5. Zusammensetzung nach Anspruch 1, des Weiteren 1,1,1,2,3,3,3,-Heptafluorpropan umfassend.

6. Zusammensetzung nach Anspruch 5, des Weiteren 5 Gewichtsprozent bis 15 Gewichtsprozent 1,1,1,2,3,3,3,-Heptafluorpropan umfassend.

7. Zusammensetzung nach Anspruch 6, umfassend:
15 Gewichtsprozent bis 18 Gewichtsprozent Pentafluorethan;
70 Gewichtsprozent bis 75 Gewichtsprozent 1,1,1,2-Tetrafluorethan;
1 Gewichtsprozent bis 3 Gewichtsprozent n-Butan;
0,5 bis 2 Gewichtsprozent n-Pentan; und
5 Gewichtsprozent bis 15 Gewichtsprozent 1,1,1,2,3,3,3-Heptafluorpropan.

8. Zusammensetzung nach Anspruch 6, umfassend:
15 Gewichtsprozent bis 17 Gewichtsprozent Pentafluorethan;
70 Gewichtsprozent bis 73 Gewichtsprozent 1,1,1,2-Tetrafluorethan;
1 Gewichtsprozent bis 3 Gewichtsprozent n-Butan;
0,5 Gewichtsprozent bis 2 Gewichtsprozent n-Pentan; und
9 Gewichtsprozent bis 11 Gewichtsprozent 1,1,1,2,3,3,3-Heptafluorpropan.

9. Zusammensetzung nach Anspruch 1, umfassend eine azeotropähnliche Zusammensetzung umfassend:
13 Gewichtsprozent bis 20 Gewichtsprozent Pentafluorethan;
70 Gewichtsprozent bis 80 Gewichtsprozent 1,1,1,2-Tetrafluorethan;
1 Gewichtsprozent bis 3 Gewichtsprozent n-Butan; und
0,5 bis 2 Gewichtsprozent n-Pentan.

10. Zusammensetzung nach Anspruch 5 umfassend eine azeotropähnliche Zusammensetzung umfassend:
15 Gewichtsprozent bis 18 Gewichtsprozent Pentafluorethan;
70 Gewichtsprozent bis 75 Gewichtsprozent 1,1,1,2-Tetrafluorethan;
1 Gewichtsprozent bis 3 Gewichtsprozent n-Butan;
0,5 bis 2 Gewichtsprozent n-Pentan; und
5 Gewichtsprozent bis 15 Gewichtsprozent 1,1,1,2,3,3,3-Heptafluorpropan.

11. Verfahren zur Erzeugung von Kühlung, umfassend das Kondensieren der Zusammensetzung nach Anspruch 1 und daraufhin das Verdampfen der Zusammensetzung in der Nähe eines zu kühlenden Körpers.

12. Verfahren zur Erzeugung von Wärme, umfassend das Kondensieren der Zusammensetzung nach Anspruch 1 in der Nähe eines zu erwärmenden Körpers und daraufhin das Verdampfen der Zusammensetzung.

13. Verfahren zum Wiederaufladen eines Kühlsystems, das ein zu ersetzendes Kühlmittel und ein Schmiermittel umfasst, wobei das Verfahren Folgendes umfasst:
das Entfernen des zu ersetzenden Kühlmittels aus dem Kühlsystem, während ein wesentlicher Anteil des Schmiermittels in dem System zurückgehalten wird; und
das Einführen in das Kühlsystems einer Kühlmittelzusammensetzung nach Anspruch 1.

14. Wärmeaustauschsystem umfassend eines Zusammensetzung nach Anspruch 1, wobei das System aus der Gruppe ausgewählt ist bestehend aus Klimaanlagen, Tiefkühlern, Kühlschränken, Wasserkühlern, begehbaren Kühlvorrichtungen, Wärmepumpen und mobile Kühlschrank- und Klimaanlageanwendungen und Kombinationen davon.

## Revendications

1. Composition comprenant:
13 pour cent en poids à 20 pour cent en poids de pentafluoroéthane;
70 pour cent en poids à 80 pour cent en poids de 1,1,1,2-tétrafluoroéthane; et
1 pour cent en poids à 6 pour cent en poids total d'une combinaison d'hydrocarbures constitués de *n*-butane et de *n*-pentane.

2. Composition selon la revendication 1, dans laquelle ladite combinaison d'hydrocarbures comprend de 1 pour cent en poids à 3 pour cent en poids de *n*-butane; et 0,5 pour cent en poids à 2 pour cent en poids de *n*-pentane.

3. Composition selon la revendication 1, comprenant:
15 pour cent en poids à 20 pour cent en poids de pentafluoroéthane;
75 pour cent en poids à 80 pour cent en poids de 1,1,1,2-tétrafluoroéthane;
1 pour cent en poids à 3 pour cent en poids de *n*-butane; et
0,5 pour cent en poids à 2 pour cent en poids de *n*-pentane.

4. Composition selon la revendication 1, comprenant:
17 pour cent en poids à 20 pour cent en poids de pentafluoroéthane;
77 pour cent en poids à 80 pour cent en poids de 1,1,1,2-tétrafluoroéthane;
1 pour cent en poids à environ 3 pour cent en poids de *n*-butane; et
0,5 à 2 pour cent en poids de *n*-pentane.

5. Composition selon la revendication 1, comprenant en outre du 1,1,1,2,3,3,3-heptafluoropropane.

6. Composition selon la revendication 5, comprenant en outre 5 pour cent en poids à 15 pour cent en poids de 1,1,1,2,3,3,3-heptafluoropropane.

7. Composition selon la revendication 6, comprenant:
15 pour cent en poids à 18 pour cent en poids de pentafluoroéthane;
70 pour cent en poids à 75 pour cent en poids de 1,1,1,2-tétrafluoroéthane;
1 pour cent en poids à 3 pour cent en poids de *n*-butane;
0,5 à 2 pour cent en poids de *n*-pentane; et
5 pour cent en poids à 15 pour cent en poids de 1,1,1,2,3,3,3-heptafluoropropane.

8. Composition selon la revendication 6, comprenant:
15 pour cent en poids à 17 pour cent en poids de pentafluoroéthane;
70 pour cent en poids à 73 pour cent en poids de 1,1,1,2-tétrafluoroéthane;
1 pour cent en poids à 3 pour cent en poids de *n*-butane;
0,5 pour cent en poids à 2 pour cent en poids de *n*-pentane; et
9 pour cent en poids à 11 pour cent en poids de 1,1,1,2,3,3,3-heptafluoropropane.

9. Composition selon la revendication 1, comprenant une composition de type azéotrope comprenant:
13 pour cent en poids à 20 pour cent en poids de pentafluoroéthane;
70 pour cent en poids à 80 pour cent en poids de 1,1,1,2-tétrafluoroéthane;
1 pour cent en poids à 3 pour cent en poids de *n*-butane; et
0,5 à 2 pour cent en poids de *n*-pentane.

10. Composition selon la revendication 5, comprenant une composition de type azéotrope comprenant:
15 pour cent en poids à 18 pour cent en poids de pentafluoroéthane;
70 pour cent en poids à 75 pour cent en poids de 1,1,1,2-tétrafluoroéthane;
1 pour cent en poids à 3 pour cent en poids de *n*-butane;
0,5 à 2 pour cent en poids *n*-pentane; et
5 pour cent en poids à 15 pour cent en poids 1,1,1,2,3,3,3-heptafluoropropane.

11. Procédé pour produire le refroidissement comprenant la condensation de la composition selon la revendication 1 et par la suite l'évaporation de ladite composition à proximité d'un corps à refroidir.

12. Procédé de production de chaleur comprenant la condensation de la composition selon la revendication 1 à proximité d'un corps à chauffer et par la suite l'évaporation de ladite composition.

13. Procédé de recharge d'un système de refroidissement qui contient un agent réfrigérant à remplacer et un lubrifiant, ledit procédé comprenant:
l'élimination de l'agent réfrigérant à remplacer du système de refroidissement tout en conservant une portion substantielle du lubrifiant dans ledit système; et
l'introduction dans le système de refroidissement d'une composition réfrigérante selon la revendication 1.

14. Système d'échange de chaleur comprenant une composition selon la revendication 1, dans lequel ledit système est sélectionné parmi le groupe constitué des dispositifs de conditionnement d'air, des dispositifs de congélation, des réfrigérateurs, des refroidisseurs d'eau, des dispositifs refroidissants ambulants, des pompes à chaleur, et des applications de conditionnement d'air et de réfrigérateur mobile et de leurs combinaisons.
